# EUROPEAN PATENT APPLICATION

(11) **EP 1 806 121 A1**
(43) Date of publication of application: **11.07.2007**
(21) Application number: 05795223.6
(22) Date of filing: 13.10.2005
(51) Int. Cl.: A61K 6/00

(54) **ANTI-GRAYING PREPARATION**

(30) Priority: 13.10.2004 JP 2004298442
(71) Applicant: Shiseido Company, Limited, Chuo-ku Tokyo 104-8010 (JP)
(72) Inventor: IDETA, Ritsuro SHISEIDO RES. CTR.(SHIN-YOKOHAMA), Kanagawa 2248558 (JP); NAKAZAWA, Yosuke SHISEIDO RES. CTR.(SHIN-YOKOHAMA), Kanagawa 2248558 (JP); IWAKI, Haruhi SHISEIDO RES. CTR.(SHIN-YOKOHAMA), Kanagawa 2248558 (JP); ISHINO, Akihiro SHISEIDO RES. CTR.(SHIN-YOKOHAMA), Kanagawa 2248558 (JP); TAJIMA, Masahiro SHISEIDO RES. CTR.(SHIN-YOKOHAMA), Kanagawa 2248558 (JP)
(74) Representative: Santarelli
(86) International application number: PCT/JP2005/019251
(87) International publication number: WO 2006/041204

(57) **Abstract**

An anti-hair graying agent containing, as an active ingredient, a substance having a superior anti-hair graying action, for example, aminocyclohexane carboxylic acid, aminocyclohexane carboxylic acid esters such as aminocyclohexane carboxylic acid ethyl ester, aminocyclohexane derivatives such as aminocyclohexanol, and piperidinecarboxylic acid, piperidinecarboxylic acid esters such as piperidinecarboxylic acid ethyl ester, and piperidine derivatives such as hydroxypiperidine is provided.

## Description

### TECHNICAL FIELD

The present invention relates to an anti-hair graying agent having an aminocyclohexane derivative or piperidine derivative as the active ingredient thereof.

### BACKGROUND ART

The melanin pigment determining the color of hair is biosynthesized from tyrosine by the melanosome in the melanocyte existing in upper portion of the matrix cell and supplied to the hair. Hair graying is believed to be due to aging or stress etc. resulting in, for example, a decrease of melanocytes or melanosomes or abnormalities in these cells or organs resulting in a drop in the amount or activity of tyrosinase, inhibition of transport of melanosomes etc., but the overall mechanism is still unclear.

As compounds having an anti-hair graying action which prevent hair graying or improve hair graying, many compounds have been proposed up to now. For example, it has been reported that Japanese pepper extract has this kind of anti-hair graying action (see Japanese Patent Publication (A) No. 11-5720). However, there is still no substance which possesses a sufficiently satisfactory effect. Further, if considering the complexity of the mechanism of occurrence of hair graying, enrichment of the active ingredients possessing a superior anti-hair graying action is also desired.

The aminocyclohexane derivatives and piperidine derivatives according to the present invention are known compounds, but their anti-hair graying effects have not been known up to now. They were confirmed for the first time by the present inventors.

### DISCLOSURE OF THE INVENTION

The present invention was made in consideration of the above situation and the its object thereof is to provide an anti-hair graying agent comprising, as an active ingredient, a substance having a superior anti-hair graying action.

The inventors engaged in repeated intensive studies to solve the above-mentioned problems and, as a result, discovered that specific aminocyclohexane derivatives and piperidine derivatives have superior anti-hair graying actions, whereby the above problems were able to be solved and, thus, completed the present invention.

That is, the present invention provides an anti-hair graying agent comprising, as an active ingredient, an aminocyclohexane derivative having the formula (1) : 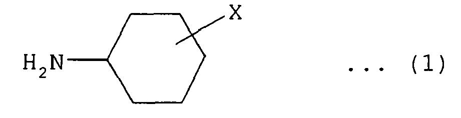 wherein X represents a -COOR group, where R represents an alkyl group or hydrogen atom, or an OH group, or a piperidine derivative having the formula (2): 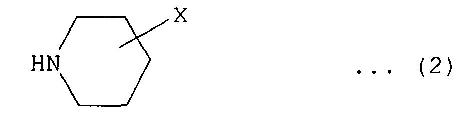 wherein X represents a -COOR group, where R represents an alkyl group or hydrogen atom, or an OH group.

The above-mentioned aminocyclohexane derivative may be a 4-aminocyclohexane carboxylic acid derivative having the formula (3): 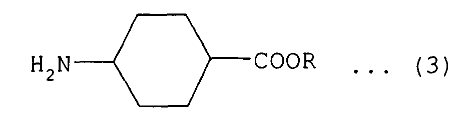 wherein R represents an alkyl group or hydrogen atom. The above-mentioned 4-aminocyclohexane carboxylic acid derivative may be 4-aminocyclohexane carboxylic acid.

Further, the above-mentioned aminocyclohexane derivative may be a 1-amino-1-cyclohexane carboxylic acid derivative having the formula (4): 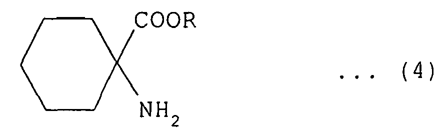 wherein R represents an alkyl group or hydrogen atom. The above-mentioned 1-amino-1-cyclohexane carboxylic acid derivative may be 1-amino-1-cyclohexane carboxylic acid.

Further, the above-mentioned aminocyclohexane derivative may be a 2-aminocyclohexane carboxylic acid derivative having the formula (5): 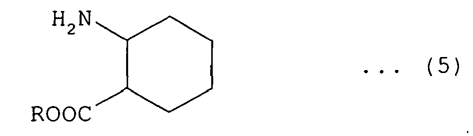 wherein R represents an alkyl group or hydrogen atom. The above-mentioned 2-aminocyclohexane carboxylic acid derivative may be 2-aminocyclohexane carboxylic acid.

Further, the above-mentioned aminocyclohexane derivative may be a 3-aminocyclohexane carboxylic acid derivative having the formula (6): 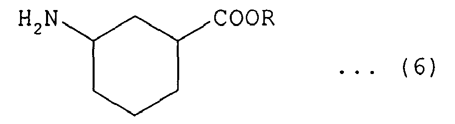 wherein R represents an alkyl group or hydrogen atom. The above-mentioned 3-aminocyclohexane carboxylic acid derivative may be 3-aminocyclohexane carboxylic acid.

Further, the above-mentioned aminocyclohexane derivative in the present invention may be 4-aminocyclohexanol having the formula (7):

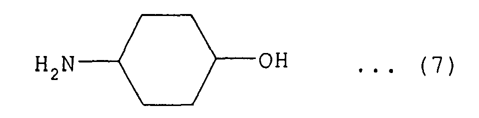

Further, the above-mentioned piperidine derivative in the present invention may be a piperidine carboxylic acid derivative having the formula (8): 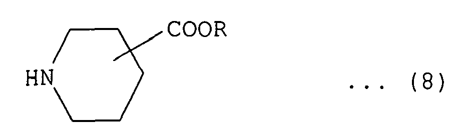 wherein R represents an alkyl group or hydrogen atom. The above-mentioned piperidine carboxylic acid derivative may be 4-piperidine carboxylic acid.

Furthermore, the above-mentioned piperidinecarboxylic acid derivative may be 4-piperidine carboxylic acid ethyl ester.

Further, the piperidine derivative in the present invention may be 4-hydroxypiperidine having the formula

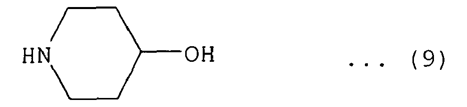

The active ingredients of the present invention constituted by the aminocyclohexane derivative having formula (1) and the piperidine derivative having formula (2) have a superior anti-hair graying action and are useful as an anti-hair graying agent. By applying the anti-hair graying agent to the human scalp, it is possible to prevent or improve hair graying.

### BRIEF DESCRIPTION OF THE DRAWINGS

The present invention will now be specifically described referring to the drawings.

FIG. 1 shows the melanocyte proliferation stimulation effect of compound 1 (i.e., 4-aminocyclohexane carboxylic acid), which is an example of the active ingredient of the present invention.

FIG. 2 shows the melanocyte proliferation stimulation effect of compound 2 (i.e., 4-piperidine carboxylic acid), which is an example of the active ingredient of the present invention.

FIG. 3 shows the melanocyte proliferation stimulation effect of compound 3 (i.e., 4-piperidine carboxylic acid ethyl ester), which is an example of the active ingredient of the present invention.

FIG. 4 shows the melanocyte proliferation stimulation effect of compounds 4 to 7, which are examples of the active ingredients of the present invention.

FIG. 5 shows the melanocyte proliferation stimulation effect of compound 8, which is an example of the active ingredient of the present invention.

FIG. 6 shows the melanocyte migration stimulation effect of an example of compound 1 (i.e., 4-aminocyclohexane carboxylic acid), which is the active ingredient of the present invention.

FIG. 7 shows the melanocyte migration stimulation effect of an example of compound 2 (i.e., 4-piperidine carboxylic acid), which is the active ingredient of the present invention.

FIG. 8 shows the melanocyte migration stimulation effect of an example of compound 3 (i.e., 4-piperidine carboxylic acid ethyl ester), which is the active ingredient of the present invention.

### BEST MODE FOR CARRYING OUT THE INVENTION

The best mode for carrying out the present invention will now be described. Note that, in the description and claims, the singular form shall be deemed to include the plural form, unless the singular form is clear from the context.

The aminocyclohexane derivative, which is the active ingredient of the present invention, having the formula (1) : 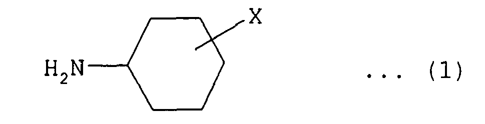 wherein X is a -COOR group or an OH group. The R in the -COOR group is an alkyl group or hydrogen atom. As the alkyl group, a C₁ to C₄ alkyl group is preferable. Particularly, an ethyl group is preferable.

The bond positions of the amino group of the aminocyclohexane derivative may be any of the 1-position, 2-position, 3-position, and 4-position with respect to the bond position of X. For example, the 4-aminocyclohexane carboxylic acid derivative having the formula (3): 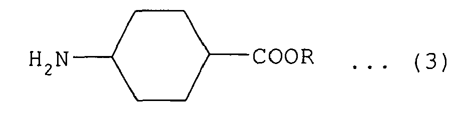 the 1-amino-1-cyclohexane carboxylic acid derivative having formula (4): 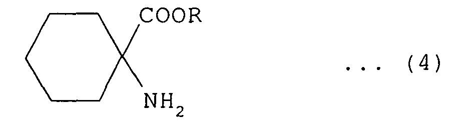 the 2-aminocyclohexane carboxylic acid derivative having formula (5): 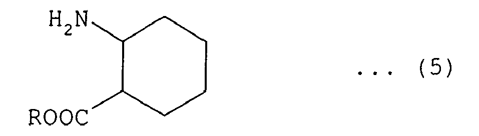 the 3-aminocyclohexane carboxylic acid derivative having formula (6): 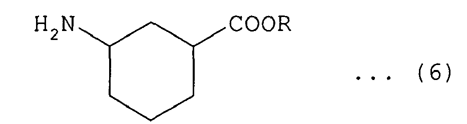 and the 4-aminocyclohexanol having formula (7): 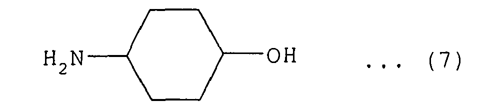 may be mentioned.

Further, the R in each of the formulae of the formulae (3) to (6) is an alkyl group or hydrogen atom, which is the same as in the above.

In the aminocyclohexane derivative in the present invention, X is preferably a -COOH group and an OH group.

Further, the above-mentioned aminocyclohexane derivative has an asymmetric center. In the present invention, when there is an optical isomer based on the asymmetric carbon atom or another isomer, it is possible to use each isomer and their mixtures. For example, in the case of 4-aminocyclohexane carboxylic acid, an isomer where an amino group and a carboxylic acid group at the cis-positions across from a cyclohexane ring may be illustrated.

As the aminocyclohexane derivative in the present invention, specifically, for example, 4-aminocyclohexane carboxylic acid, 1-aminocyclohexane carboxylic acid, 2-aminocyclohexane carboxylic acid, 3-aminocyclohexane carboxylic acid, 4-aminocyclohexane carboxylic acid ethyl ester, 1-aminocyclohexane carboxylic acid ethyl ester, 2-aminocyclohexane carboxylic acid ethyl ester, 3-aminocyclohexane carboxylic acid ethyl ester, 4-aminocyclohexane carboxylic acid butyl ester, 2-aminocyclohexane carboxylic acid butyl ester, 4-aminocyclohexane carboxylic acid methyl ester, 3-aminocyclohexane carboxylic acid methyl ester, 4-aminocyclohexanol, 1-aminocyclohexanol, 2-aminocyclohexanol, 3-aminocyclohexanol and the like may be mentioned.

In the active ingredient of the present invention, i.e., the piperidine derivative having the formula (2): 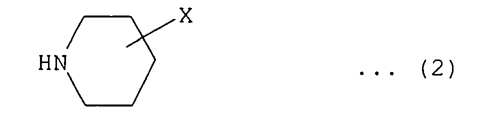 wherein X is a -COOR group or an OH group. In the case where X is a -COOR group, it is represented, as the piperidine carboxylic acid derivative having the formula (8): 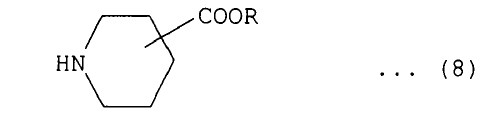 wherein R in the -COOR group in the formula (8) is an alkyl group or a hydrogen atom. The above-mentioned.alkyl group is preferably a C₁ to C₄ alkyl group. Particularly preferable is an ethyl group. The X is preferably a -COOH group, a -COOC₂H₅ group or an OH group.

Further, the bond position of X of the piperidine derivative may be any of the 1-position, 2-position, 3-position, and 4-position, but location at the 4-position is preferable.

As the piperidine derivative in the present invention, specifically, for example, 4-piperidine carboxylic acid, 1-piperidine carboxylic acid, 2-piperidine carboxylic acid, 3-piperidine carboxylic acid, 4-piperidine carboxylic acid ethyl ester, 1-piperidine carboxylic acid ethyl ester, 2-piperidine carboxylic acid ethyl ester, 3-piperidine carboxylic acid ethyl ester, 4-piperidine carboxylic acid butyl ester, 2-piperidine carboxylic acid butyl ester, 4-piperidine carboxylic acid methyl ester, 3-piperidine carboxylic acid methyl ester, 4-hydroxypiperidine having the formula (9): 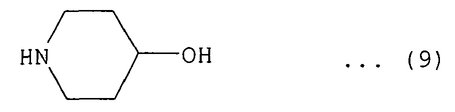 1-hydroxypiperidine, 2-hydroxypiperidine, 3-hydroxypiperidine and the like. Among these, 4-piperidine carboxylic acid, 4-piperidine carboxylic acid ethyl ester and 4-hydroxypiperidine are preferred.

The aminocyclohexane derivatives and the piperidine derivatives in the present invention may be those produced by chemical synthesis or may be those produced and extracted from microorganisms and animal and plant cells. They are not particularly limited.

The aminocyclohexane derivatives and piperidine derivatives according to the present invention, as will be proved later, have a human melanocyte proliferation stimulation effect and a human melanocyte migration stimulation effect. Due to this, if applying said compounds to the scalp, the progression of hair graying is suppressed. Consequently, said aminocyclohexane derivatives and piperidine derivatives are contained as active ingredients and prepared into anti-hair graying agents.

Further, for example, by simultaneously using a compound having the effect of maintaining or promoting the mitotic proliferation activity of follicle epithelial cells or hair growth activity of human head hair and thereby extending the hair growing phase, the activity of the hair, as a whole, is increased synergistically contributing to the effect of suppressing and improving hair graying.

In the anti-hair graying agent of the present invention, the content of the aminocyclohexane derivative or piperidine derivative is preferably 0.0005 to 20% mass in the entire amount of the anti-hair graying agent. It is more preferably 0.01 to 5% mass. If the content is too large, this is sometimes not preferred in terms of the formulation. Note that in the anti-hair graying agent of the present invention, it is possible to combine an aminocyclohexane derivative and piperidine derivative. Further, it is possible to use two types or more of each.

The anti-hair graying agent of the present invention is administered by transdermal administration by direct application to or sprinkling on the scalp skin. Further, the optimal dosage of the anti-hair graying agent of the present invention is suitably determined by the age, individual differences, symptoms, etc., but the dosage in the case of humans is usually 0.01 to 100 mg/kg, preferably 0.1 to 10 mg/kg. This quantity may be administered one time per day or divided among two to four times per day.

The anti-hair graying agent of the present invention may be produced, while suitably including, in addition to the above necessary ingredients and in a range which does not impair the effect of the present invention, other ingredients usually used for cosmetics, quasi-drugs, pharmaceuticals, etc., that is, excipients and bases, if necessary. For example, powder ingredients, liquid lipids, solid lipids, waxes, hydrocarbons, higher fatty acids, higher alcohols, esters, silicone, anionic surfactants, cationic surfactants, ampholytic surfactants, nonionic surfactants, moisturizing agents, water-soluble polymer compounds, thickening agents, skin protecting agents, UV absorbing agents, sequestering agents, lower alcohols, polyvalent alcohols, sugars, amino acids, organic amines, synthetic resin emulsions, pH adjusters, hair growth preparation agents, hormones, anti-inflammatory agents, astringents, algefacients, medications against dandruff and pruritus, skin nutrients and other medicines, vitamins, antioxidants, antioxidant aids, fragrances, water and the like may be mentioned.

Specific examples of optional combinations of the ingredients are mentioned below, but the invention is not limited to these ingredients so long as the ingredient can be normally applied to cosmetics and pharmaceuticals.

As a powder ingredient, talc, kaolin, mica, sericite, muscovite, phlogopite, synthetic mica, lepidolite, biotite, lithium mica, vermiculite, magnesium carbonate, calcium carbonate, aluminum silicate, barium silicate, calcium silicate, magnesium silicate, strontium silicate, tungstic acid metal salts, magnesium, silica, zeolite, barium sulfate, calcium sulfate (calcined gypsum), calcium phosphate, fluorine apatite, hydroxyapatite, ceramic powder, metal soap (zinc myristate, calcium palmitate, aluminum stearate), boron nitride and other inorganic powders, polyamide resin powder (nylon powder), polyethylene powder, polymethyl methacrylate powder, polystyrene powder, a copolymer resin powder of styrene and acrylic acid, benzoguanamine resin powder, polytetrafluoroethylene powder, cellulose powder and other organic powders, titanium dioxide, zinc oxide and other inorganic white pigments, iron oxide (bengara), iron titanate and other inorganic red type pigments, γ-iron oxide and other inorganic brown type pigments, yellow iron oxide, loess and other inorganic yellow type pigments, black iron oxide, carbon black, lower titanium oxide and other inorganic black type pigments, Manganese Violet, Cobalt Violet and other inorganic purple type pigments, chromium oxide, chromium hydroxide, cobalt titanate and other inorganic green type pigments, Ultramarine Blue, Prussian Blue and other inorganic blue type pigments, titanated mica, titanated bismuth oxychloride, titanated talc, colored titanated mica, bismuth oxychloride, argentine and other pearl pigments, aluminum powder, copper powder and other metal powder pigments, Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404 and other organic pigments, Red No. 3, Red No. 104, Red No. 106, Red No. 227, Red No. 230, Red No. 401, Red No. 505, Orange No. 205, Yellow No 4, Yellow No. 5, Yellow No. 202, Yellow No. 203, Green No. 3, Blue No. 1 and other zirconium, barium or aluminum lakes or other organic pigments, chlorophyll, β-carotene and other natural pigments etc. may be mentioned.

As liquid lipids, avocado oil, tsubaki oil, turtle oil, macadamia nut oil, corn oil, mink oil, olive oil, rapeseed oil, egg yolk oil, sesame oil, persic oil, wheat germ oil, sasanqua oil, castor oil, linseed oil, safflower oil, cottonseed oil, perilla oil, soybean oil, peanut oil, teaseed oil, nutmeg oil, rice bran oil, Paulownia oil, Japanese paulownia oil, jojoba oil, germ oil, triglycerin, glyceryl trioctanate, glyceryl triisopalmitate and the like may be mentioned.

As solid lipids, cocoa butter, coconut oil, horse fat, hydrogenated coconut oil, palm oil, beef fat, mutton fat, hydrogenated beef fat, palm kernel oil, pork fat, beef bone fat, Japan wax kernel oil, hydrogenated oil, beef hoot fat, Japan wax, hydrogenated castor oil and the like may be mentioned.

As waxes, beeswax, Candelilla wax, cotton wax, carnauba wax, bayberry wax, ibota wax, spermaceti, montan wax, bran wax, lanolin, kapok wax, lanolin acetate, liquid lanolin, sugarcane wax, lanolin fatty acid isopropyl, hexyl laurate, reduced lanolin, jojoba wax, hydrogenated lanolin, shellac wax, polyoxyethylene (below, abbreviated as POE), lanolin alcohol ether, POE lanolin alcohol acetate, POE cholesterol ether, polyethyleneglycol lanolin fatty acid, POE hydrogenated lanolin alcohol ether and the like may be mentioned.

As hydrocarbon oils, liquid paraffin, ozokerite, squalene, pristane, paraffin, ceresin, squalane, Vaseline, microcrystalline wax and the like may be mentioned.

As higher fatty acids, for example, lauric acid, myristic acid, palmitic acid, stearic acid, behenic acid, oleic acid, petroselinic acid, 12-hydroxystearic acid, undecylenic acid, tall oil fatty acid, isostearic acid, linolic acid, linoleic acid, eicosapentaenoic acid (EPA), docosahexaenoic acid (DHA) and the like may be mentioned.

As higher alcohols, for example, lauryl alcohol, cetyl alcohol, stearyl alcohol, behenyl alcohol, myristyl alcohol, oleyl alcohol, cetostearyl alcohol and other straight chain alcohols, monostearyl glyceryl ether (batyl alcohol), 2-decyl tetradecynol, lanolin alcohol, cholesterol, phytosterol, hexyl dodecanol, isostearyl alcohol, octyl dodecanol and other branched chain alcohols etc. may be mentioned.

As synthetic ester oils, isopropyl myristate, cetyl octanoate, octyl dodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentyl glycol dicaprate, diisostearyl malate, glyceryl di-2-heptyl undecanoate, trimethylolpropane tri-2-ethylhexanoate, trimethylolpropane triisostearate, tetra-2-pentaerythritol ethylhexanoate, glyceryl tri-2-ethylhexanoate, trimethylolpropane triisostearate, cetyl-2-ethyl hexanoate, 2-ethylhexyl palmitate, glyceryl trimyristate, glyceryl tri-2-heptyl undecanoate, castor oil fatty acid methyl ester, oleyl oleate, cetostearyl alcohol, acetoglyceride, 2-heptyl undecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, triethyl citrate and the like may be mentioned.

As silicones, for example, dimethyl polysiloxane, methylphenyl polysiloxane, methyl hydrogen polysiloxane and other chain polysiloxanes, decamethyl polysiloxane, dodecamethyl polysiloxane, tetramethyl tetrahydrogen polysiloxane and other cyclic polysiloxanes, silicone resins forming 3-dimensional network structures, silicone rubber and the like may be mentioned.

As anionic surfactants, for example, natural soap, sodium laurate, sodium palmitate and other fatty acid soaps, sodium lauryl sulfate, potassium lauryl sulfate and other higher alkyl sulfuric acid ester salts, POE-triethanolamine lauryl sulfate, sodium POE lauryl sulfate and other alkyl ether sulfuric acid ester salts, sodium lauroyl sarcosine and other N-acyl sarcosinic acids, sodium myristoylmethyl taurate, sodium N-cocoyl-N-methyl taurate, sodium laurylmethyl taurate and other higher fatty acid amide sulfonates, sodium POE oleyl ether phosphate, POE stearyl ether phosphoric acid and other phosphoric acid ester salts, sodium di-2-ethylhexyl sulfosuccinate, sodium monolauroyl monoethanolamide polyoxyethylene sulfosuccinate, sodium lauryl polypropyleneglycol sulfosuccinate and other sulfosuccinates, linear sodium dodecyl benzene sulfonate, linear triethanolamine dodecyl benzene sulfonate, linear dodecyl benzene sulfonic acid and other alkyl benzene sulfonates, monosodium N-lauroyl glutamate, disodium N-stearoyl glutamate, monosodium N-myristoyl-L-glutamate and other N-acyl glutamates, sodium hydrogenated glyceryl cocoate sulfate and other high fatty acid ester sulfuric acid ester salts, turkey red oil and other sulfated oils, POE-alkylether carboxylic acid, POE-alkylarylether carboxylate, α-olefin sulfonate, higher fatty acid ester sulfonate, secondary alcohol sulfuric acid ester salts, higher fatty acid alkylolamide sulfuric acid salts, sodium lauroyl monoethanolamide succinate, ditriethanolamine N-palmitoyl asparatate, sodium caseinate and the like may be mentioned.

As cationic surfactants, for example, stearyl trimethyl ammonium chloride, lauryl trimethyl ammonium chloride and other alkyl trimethyl ammonium salts, distearyl dimethyl ammonium dialkyl dimethyl ammonium salts, poly-(N,N'-dimethyl-3,5-methylene piperidinium) chloride, cetyl piperidinium chloride and other alkyl pyridinium salts, alkyl quaternary ammonium salts, alkyl dimethylbenzyl ammonium salts, alkyl isoquinolinium salts, dialkyl morpholium salts, POE-alkyl amines, alkyl amine salts, polyamine fatty acid derivatives, amyl alcohol fatty acid derivatives, benzalkonium chloride, benzethonium chloride and the like may be mentioned.

As ampholytic surfactants, for example, sodium 2-undecyl-N,N-N-(hydroxyethylcarboxymethyl)-2-imidazoline, 2-cocoyl-2-imidazolinium hydroxide-1-carboxyethyloxy-2-sodium salt and other imidazoline-based ampholytic surfactants, 2-heptadecyl-N-carboxymethyl-N-hydroxyethyl imidazolinium betaine, lauryl dimethyl aminoacetic acid betaine, alkyl betaine, amide betaine, sulfobetaine and other betaine-based surfactants etc. may be mentioned.

As lyophilic nonionic surfactants, for example, sorbitan monooleate, sorbitan monoisostearate, sorbitan monolaurate, sorbitan monopalmitate, sorbitan monostearate, sorbitan sesquioleate, sorbitan trioleate, diglyceryl sorbitan penta-2-ethylhexanoate, diglyceryl sorbitan tetra-2-ethylhexanoate and other sorbitan fatty acid esters, glyceryl monocotton seed oil fatty acid, glyceryl monoerucate, glyceryl sesquioleate, glyceryl monostearate, glyceryl α,α'-oleate pyroglutamate, glyceryl monostearate malate and other glyceryl polyglyceryl fatty acids, propylene glycol monostearate and other propylene glycol fatty acid esters, hydrogenated castor oil derivatives, glyceryl alkyl ethers and the like may be mentioned.

As hydrophilic nonionic surfactants, for example, POE-sorbitan monooleate, POE-sorbitan monostearate, POE-sorbitan monooleate, POE-sorbitan tetraoleate and other POE sorbitan fatty acid esters, POE-sorbitol monolaurate, POE-sorbitol monooleate, POE-sorbitol pentaoleate, POE-sorbitol monostearate and other POE-sorbitol fatty acid esters, POE-glyceryl monostearate, POE-glyceryl monoisostearate, POE-glyceryl triisostearate and other POE-glycerin fatty acid esters, POE-monooleate, POE-distearate, POE-monodioleate, ethylene glycol distearate and other POE fatty acid esters, POE-lauryl ether, POE-oleyl ether, POE-stearyl ether, POE-behenyl ether, POE-2-octyl dodecyl ether, POE-cholestanol ether and other POE alkyl ethers, POE-octylphenyl ether, POE-nonylphenyl ether, POE-dinonylphenyl ether and other POE alkyl phenyl ethers, pluaronic type resin such as Pluronic^{™} POE-POP-cetyl ether, POE-POP-2-decyl tetradecyl ether, POE-POP-monobutyl ether, POE-POP-hydrogenated lanolin, POE-POP-glyceryl ether and other POE-POP-alkyl ethers, Tetronic and other tetra POE-tetra POP-ethylene diamine condensates, POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, POE-hydrogenated castor oil malate and other POE-hydrogenated castor oil derivatives; POE-sorbitol beeswax and other POE-beeswax-lanolin derivatives, coconut fatty acid diethanolamide, laurate monoethanol amide laurate, fatty acid isopropanol amide and other alkanol amides, POE-propylene glycol fatty acid esters, POE-alkyl amines, POE-fatty acid amides, sucrose fatty acid esters, POE-nonylphenyl formaldehyde condensates, alkylethoxy dimethylamine oxides, trioleyl phosphoric acids and the like may be mentioned.

As moisturizing agents, for example, polyethylene glycol, propylene glycol, glycerin, 1,3-butylene glycol, xylitol, sorbitol, maltitol, chondroitin sulfate, hyaluronic acid, mucoitin sulfuric acid, charonic acid, atelocollagen, cholesteryl-12-hydroxy stearate, sodium lactate, billiary acid salt, dl-pyrrolidone carboxylic acid salt, short chain soluble collagen, diglycerin (EO) PO adduct, chestnut rose extract, yarrow extract, sweet clover extract and the like may be mentioned.

As natural water soluble polymers, for example, gum arabic, tragacanth gum, galactan, guar gum, carob gum, karaya gum, carrageenan, pectin, agar, quince, seaweed (brown algae extract), starch (rice, corn, potato, wheat), glycyrrhizinic acid and other plant-based polymers, xanthan gum, dextran, succinoglycan, brulant and other microorganism-based polymers, collagen, casein, albumin, gelatin and other animal-based polymers may be mentioned.

As semi-synthetic water soluble polymers, for example, carboxymethyl starch, methylhydroxypropyl starch and other starch-based polymers, methyl cellulose, nitrocellulose, ethyl cellulose, methyl hydroxypropyl cellulose, hydroxyethyl cellulose, cellulose sodium sulfate, hydroxypropyl cellulose, sodium carboxymethyl cellulose (CMC), crystal cellulose, cellulose powder and other cellulose-based polymers, sodium alginate, propylene glycol alginate esters and other alginic acid-based polymers etc. may be mentioned.

As synthetic water soluble polymers, for example, polyvinyl alcohol, polyvinyl methyl ether, polyvinyl pyrrolidone, carboxyl vinyl polymer (Carbopol) and other vinyl-based polymers; polyethylene glycol 20,000, 40,000, 60,000 and other polyoxyethylene-based polymers, polyoxyethylene polyoxypropylene copolymers copolymer-based polymers, sodium polyacrylate, polyethyl acrylate, polyacryl amide and other acryl-based polymers, polyethylene imines, cationic polymers and the like may be mentioned.

As thickening agents, for example, gum arabic, carrageenan, karaya gum, tragacanth gum, carob gum, quince seed (Cydonia oblonga), casein, dextrin, gelatin, sodium pectinate, sodium arachidonate, methyl cellulose, ethyl cellulose, CMC, hydroxyethyl cellulose, hydroxypropyl cellulose, PVA, PVM, PVP, sodium polyacrylate, carboxyl vinyl polymer, locustbean gum, guar gum, tamarind gum, cellulose dialkyl dimethylammonium sulfate, xanthan gum, aluminum magnesium silicate, bentonite, hectorite, laponite, silicic anhydride and the like may be mentioned.

As UV absorbants, for example, para-amino benzoic acid (below, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester and other benzoic acid-based UV absorbants, homomentyl-N-acetyl anthranilate and other anthranilic acid-based UV absorbants, amyl salicylate, mentyl salicylate, homomentyl salicylate, octyl salicylate, phenyl salicylate, benzil salicylate, p-isopropanol phenyl salicylate and other salicylic acid-based UV absorbants, octyl cinnamate, ethyl 4-isopropyl cinnamate, methyl 2,5-diisopropyl cinnamate, ethyl 2,4-diisopropyl cinnamate, methyl 2,4-diisopropyl cinnamate, propyl-p-methoxy cinnamate, isopropyl p-methoxy cinnamate, iso-amyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl a-cyano-β-phenyl cinnamate, 2-ethylhexyl-α-cyano-β-phenyl cinnamate, glyceryl mono-2-ethylhexanoyl-diparamethoxy cinnamate and other cinnamic acid-based UV absorbants, 3-(4'-methylbenzylidene)-d,l-camphor, 3-benzylidene-d-,1-camphor; urocanate, ethyl urocanate, 2-phenyl-5-methyl benzoxazole, 2,2'-hydroxy-5-methylphenyl benzotriazole, 2-(2'-hydroxy-5'-t-octylphenyl)benzotriazole, 2-(2'-hydroxy-5'-methylphenyl benzotriazole, dibenzaladine, dianisoylmethane, 4-methoxy-4'-t-butyl dibenzoyl methane, 5-(3,3-dimethyl-2-norbornylidene)-3-pentane-2-one and the like may be mentioned.

As metal ion sequestering agents, for example, 1-hydroxyethane-1, 1-diphosphonic acid, 1-hydroxyethane-1,1-phosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid and the like may be mentioned.

As lower alcohols, for example, methanol, ethanol, propanol, isopropanol, isobutyl alcohol, t-butyl alcohol and the like may be mentioned.

As polyhydric alcohols, for example, ethylene glycol, propylene glycol, trimethylene glycol, 1,2-butyleneglycol, 1,3-butylene glycol, tetramethylene glycol, 2,3-butylene glycol, pentamethylene glycol, 2-butene-1,4-diol, hexylene glycol, octylene glycol and other dihydric alcohols, glycerin, trimethylolpropane, 1,2,6-hexanetriol and other trihydric alcohols, pentaerythritol and other tetrahydric alcohols, xylitol and other pentahydric alcohols, sorbitol, mannitol and other hexahydric alcohols, diethylene glycol, dipropylene glycol, triethylene glycol, polypropylene glycol, tetraethylene glycol, diglycerin, polyethylene glycol, triglycerin, tetraglycerin, polyglycerin and other polyhydric alcohol polymers, ethylene glycol monomethyl ether, ethylene glycol monoethyl ether, ethylene glycol monobutyl ether, ethylene glycol monophenyl ether, ethylene glycol monohexyl ether, ethylene glycol mono 2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether and other dihydric alcohol alkylethers, diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether and other dihydric alcohol alkyl ethers, ethylene glycol monomethyl ether acetate, ethylene glycol monoethyl ether acetate, ethylene glycol monobutyl ether acetate, ethylene glycol monophenyl ether acetate, ethylene glycol di-PBSA, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate and other dihydric alcohol ether esters, xylyl alcohol, selachyl alcohol, batyl alcohol and other glycerin mono alkyl ethers, sorbitol, maltitol, maltotriose, mannitol, sucrose, erythritol, glucose, fructose, starch decomposition sugars, maltose, xylitose, starch amilolisis sugar reduced alcohols and other sugar alcohols, glysolid, tetrahydrofurfuryl alcohol, POE-tetrahydrofurfuryl alcohol, POP-butyl ether, POP-POE-butyl ether, tripolyoxypropylene glycerin ether, POP-glycerin ether, POP-glycerin ether phosphoric acid, POP-POE-pentane erythritol ether and the like may be mentioned.

As monosaccharides, for example, D-glyceryl aldehyde, dihydroxyacetone and other trioses, D-erythrose, D-erythrulose, D-threose, erythritol and other tetroses, L-arabinose, D-xylose, L-lyxose, D-arabinose, D-ribose, D-ribulose, D-xylulose, L-xylulose and other pentoses, D-glucose, D-talose, D-Psicose, D-galactose, D-fructose, L-galactose, L-mannose, D-tagatose and other hexoses, aldoheptose, heptrose and other heptoses, octulose and other octoses, 2-dioxy-D-ribose, 6-dioxy-L-galactose, 6-dioxy-L-mannose and other dioxysugars, D-glucosamine, D-galactosamine, sialic acid, amino uronic acid, muramic acid and other amino sugars, D-glucuronic acid, D-mannuronic acid, L-gluronic acid, D-galacturonic acid, L-iduronic acid and other uronic acids etc. may be mentioned.

As oligiosaccharide, for example, sucrose, gunchianose, umbelliferose, lactose, planteose, isolignoses, α,α-trehalose, raffinose, lignoses, umbellicine, stachyose verbascoses and the like may be mentioned.

As polysaccharides, for example, cellulose, quince seed, chondroitin sulfate, starch, galactan, dermatan sulfate, glycogen, gum arabic, heparan sulfate, hyaluronic acid, traganth gum, keratan sulfate, chondroitin, xanthan gum, mucoitin sulfuric acid, guar gum, dextran, kerato sulfate, locustbean gum, succinoglucan, charonic acid and the like may be mentioned.

As amino acids, for example, glycine, alanine, valine, leucine, isoleucine, serine, threonine, tryptophan, cystine, cysteine, methionine, proline, hydroxyproline and other neutral amino acids, asparatic acid, glutamic acid, asparagine, glutamine and other acidic amino acids, and arginine, histidine, lysine, hydroxylysine and other alkaline amino acids may be mentioned. Further, as the amino acid derivatives, for example, sodium acyl sarcosine (sodium lauroyl sarcosinate), acylglutamate salt, acyl β-alanine sodium, glutathione, pyrrolidone carboxylic acid and the like may be mentioned.

As organic amines, for example, monoethanolamine, diethanolamine, triethanolamine, morpholine, triisopropanolamine, 2-amino-2-methyl-1,3-propane-diol, 2-amino-2-methyl-1-propanol and the like may be mentioned.

As synthetic resin emulsions, for example, acrylic resin emulsions, ethyl polyacrylate emulsions, acryl resin liquid, polyacrylic alkyl ester emulsions, polyvinyl acetate resin emulsions and the like may be mentioned.

As pH adjusters, for example, lactic acid-sodium lactate, citric acid-sodium citrate and other buffering agents may be mentioned.

As hair growth agents, for example, Evening Star (Swertia japonica) extract, cepharanthine, Vitamin E and its derivatives, γ-orizanol, capsicum extract, ginger root extract, Cantharides tincture, nicotinic acid benzyl ester, adenosine, minoxidil, placenta extract, allantoin, photosensitive element 301 and the like may be mentioned. As hormones, for example, estradiol, ethynyl estradiol, estrone, cortisone, hydrocortisone, prednisone, prednisolone and the like may be mentioned. As anti-inflammatory agents, for example, β-glycyrrhizinate, glycyrrhizinate derivatives, azulene, ε-amino caproic acid and the like may be mentioned. As astringents, for example, zinc oxide, zinc sulfate, allantoin hydroxy aluminum, aluminum chloride, aluminum sulfate, zinc phenolsulfonate, tannin acid, citric acid, lactic acid, while as refrigerants, menthol, camphor and the like may be mentioned. In addition, there may be provided as medications against dandruff and itching, for example, diphenhydramine hydrochloride, chlorpheniramine malate, glycyrrhizinate derivatives and other antihistamines, salicylic acid, sulfur, resorcin, selenium sulfide and other keratin exfoliators, trichlorocarbanide, zinc pyrithione, benzalkonium chloride, benzethonium chloride, chlorohexidine, cypress thiol, phenol, isopropylmethylphenol and other bactericides, and diphendramine hydrochloride and other anti-itching agents etc. may be mentioned.

As vitamins, for example, vitamins A, B1, B2, B6, E, and their derivatives, pantothenic acid and its derivatives, biotin and the like may be mentioned. As antioxidants, for example, tocopherols, dibutyl hydroxytoluene, butyl hydroxyanisole, gallate esters and the like may be mentioned. As auxillary antioxidants, for example, phosphoric acid, citric acid, ascorbic acid, maleic acid, malonic acid, succinate, fumaric acid, cephalin, hexametaphosphate, phytic acid, ethylene diamine tetraacetic acid and the like may be mentioned.

The anti-hair graying agent according to the present invention may be produced by an ordinary method in accordance with the form. This form is not particularly limited so long as the effect of the present invention can be exhibited. For example, it may be a tonic or other solution, a milky lotion, cream or other emulsion, an ointment, dispersion, gel, aerosol, mousse or any other form. Further, the type of product may also be a pharmaceutical, quasi-drug or cosmetic for hair care aimed at preventing or reversing hair graying. For example, it may be a scalp treatment agent, hair tonic, shampoo, rinse, hair pack, lotion, conditioner, scalp treatment or any other type.

### EXAMPLES

Specific Examples are now provided to further explain the present invention, but the present invention is not limited to these Examples. Note that the amounts blended and concentrations are indicated by mass% unless specifically otherwise indicated.

### Example 1

### Test Example 1: Test of Human Melanocyte Proliferation Stimulation Effect

### A. Cell Preparation

Commercially available human melanocytes were used for a test of the cell proliferation stimulation effect was performed. Specifically, normal human melanocytes (Cascade Co.) cultured according to the ordinary method in a medium comprised of a M154s medium (Cascade Co.) to which HMGS (Cascade Co.) was added were sown using a phosphate buffer containing 0.05% trypsin and EDTA above a type I collagen coat plate with 96 wells (Iwaki Glass) at a density of 3,000 cells/well.

### B. Preparation of Test Medium

### (1) Preparation of Test Substance-Containing Medium

Additive factors (bovine hypophysis pituitary gland extract 0.20%, bovine fetal serum 0.50%, human recombinant fibroblast growth factor (bFGF) 0.1 ng/ml, hydrocortisone 5×10⁻⁷ M, insulin 5 µg/ml, transferrin 5 µg/ml, 12-tetradecanoylphorbol-13-acetate 10 ng/ml and heparin 3 µg/ml) corresponding to HMGS were added to the M154s medium to obtain the test medium. Only the concentration of bFGF was made 1/30 of the prescribed amount. The test substance (dissolved in ethanol) was added to the test medium to obtain the test substance-containing medium. The concentration of ethanol in the medium was adjusted to give a final concentration of 0.1%.

### (2) Preparation of Control Media

Negative control (no test substance added): Ethanol was added to the test medium to a final concentration of 0.1%.

Positive control: Recombinant human stem cell proliferation factor (SCF) was dissolved in a 10mM acetic acid solution. This was added to the above medium to give a final concentration of 50 ng/ml.

### C. Addition of Test Medium

The medium was removed from the wells of the melanocytes sown the previous day as in A. Instead, the test substance-containing medium or control culture media prepared in B were added (N=8). After 4 days of culture (culture conditions: 37°C, 5% CO₂), the degrees of cell proliferation were measured.

### D. Measurement of Cell Proliferation

1 mg/ml of Hoechst 33342 (Sigma Co.) was added to each well of the aforementioned C in an amount of 1/100 of the medium. The cells were incubated at 37°C (5% CO₂) for 15 minutes, then fluorescent intensity of each well was measured by an excitation light wavelength 355 nm and a measurement light wavelength of 460 nm. The day before measurement, the same melanocytes that were used in the measurement were sown into the empty wells of the plate in rates of 10,000 cells/well, 5,000 cells/well, 2,500 cells/well and 1,250 cells/well. Using these, a calibration curve was prepared. Based on this, the number of cells in each well was calculated.

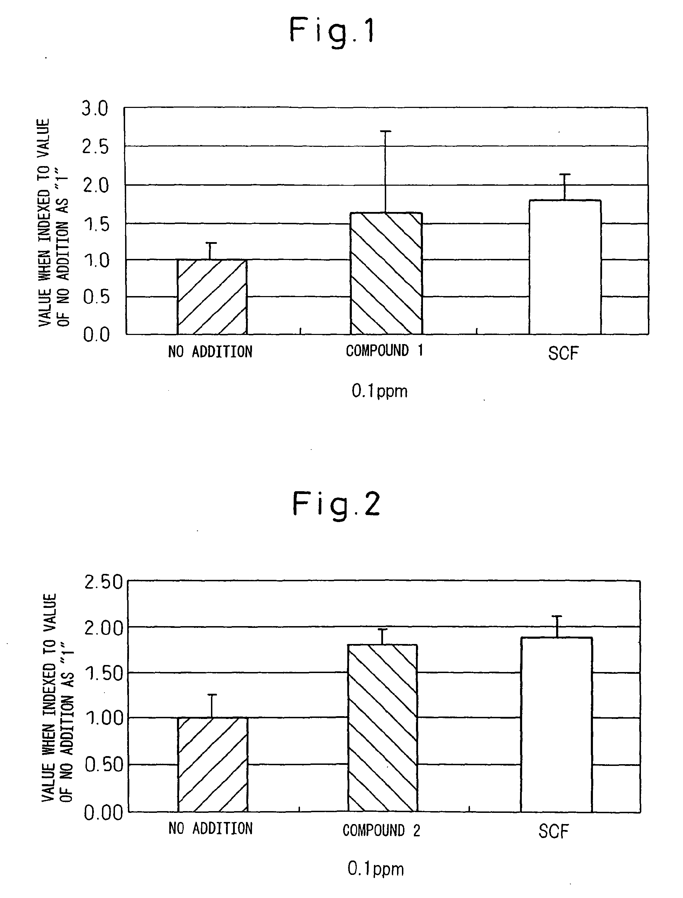

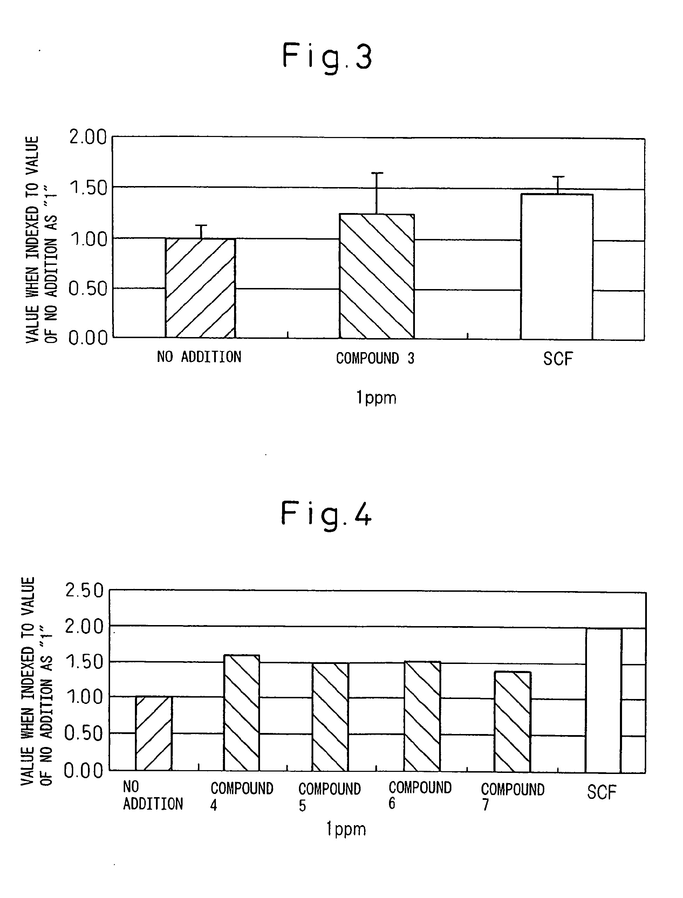

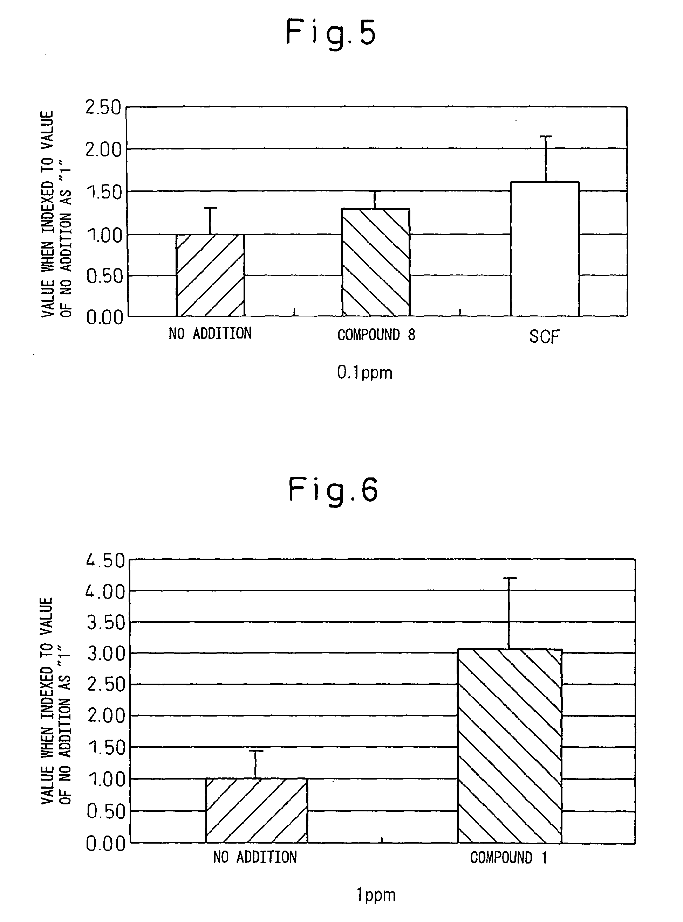

Using the average number of cells of the negative control added group as "1", the ratio of the average number of cells of each test substance added group with respect to this was calculated and used as an indicator of the melanocyte proliferation stimulation effect. The results are shown in FIGS. 1 to 5. Note that FIG. 1 shows the melanocyte proliferation stimulation effect of 4-aminocyclohexane carboxylic acid (referred to as "compound 1"), FIG. 2 shows the melanocyte proliferation stimulation effect of 4-piperidine carboxylic acid (referred to as "compound 2"), FIG. 3 shows the melanocyte proliferation stimulation effect of 4-piperidine carboxylic acid ethyl ester (referred to as "compound 3"), FIG. 4 shows the melanocyte proliferation stimulation effects of 1-aminocyclohexane carboxylic acid (referred to as "compound 4"), 2-aminocyclohexane carboxylic acid (referred to as "compound 5"), 3-aminocyclohexane carboxylic acid (referred to as "compound 6") and 4-aminocyclohexanol (referred to as "compound 7"), FIG. 5 shows the melanocyte proliferation stimulation effect of 4-hydroxypiperidine (referred to as "compound 8").

As clear from FIGS. 1 to 5, the compounds of the present invention are confirmed to have a proliferation activity of human melanocytes. It is learned that they have an anti-hair graying action based on the maintenance of the mitotic proliferation activity of the melanocytes.

### Test Example 2: Test of Human Melanocyte Migration Ability Stimulation Effect

Melanocytes of the skin are distributed in the skin at a comparatively low density, but melanocytes of the follicles are distributed at a comparatively high density around the dermal papilla. The reason is believed to be that the dermal papilla cause migration of the melanocytes of the follicles to the dermal papilla. It has been reported that an anti-hair graying activity is recognized in a substance strengthening the migration of the melanocyte by incubation with the melanocytes (see the above Japanese Patent Publication (A) No. 11-5720). Therefore, the inventors studied the compounds of the present invention for the existence of a stimulation effect on the migration ability of melanocytes.

### A. Cell Preparation

Commercially available melanocytes cultured in the same way as the above test of the proliferation stimulation effect were used.

### B. Preparation of Test Medium

(1) Test substance-containing medium: Serum free Nissui DMEM (Dulbecco Modified Eagle Medium) with the test substance diluted to double the final concentration was used.
(2) Control medium: As a negative control, the DMEM medium without the addition of the test substance was used.
(3) Preparation of migration factor containing medium Fibronectin (Sigma Co.) was diluted in a DMEM medium at a 3 µg/ml concentration. This was used as the migration factor (chemo-attractant). This migration factor-containing medium was mixed with an equal amount of the test substance-containing medium (above B-(1)), then 29 µl was distributed in each of the lower wells of a chemotaxis chamber ChemoTx (Neuroprobe Co.) A ChemoTX filter was placed on top and a top plate (upper wells) was set on top of that.

### C. Measurement of Migration Stimulation Effect

The melanocytes cultured by the above A were removed from the incubator by treatment by 0.05% trypsin and were suspended in serum free DMEM medium. The cell suspension concentration was made approximately 50,000 to 200,000 cells/ml in range. The cell suspension was equally divided and equal parts were mixed with the test substance-containing medium (B-(1)) or the control medium (B-(2)). 25 µl of this was applied to each of the upper wells.

The cells were incubated under the conditions of 37°C and 5% CO₂ overnight, then the filter was removed, carefully cleaned of not migrating cells at the top surface of the filter, fixed in 70% ethanol for 7 minutes, then rinsed and air dried. The filter was stained by 5% Giemsa stain solution, and the number of cells migrating to the bottom surface of the filter was counted by a microscope.

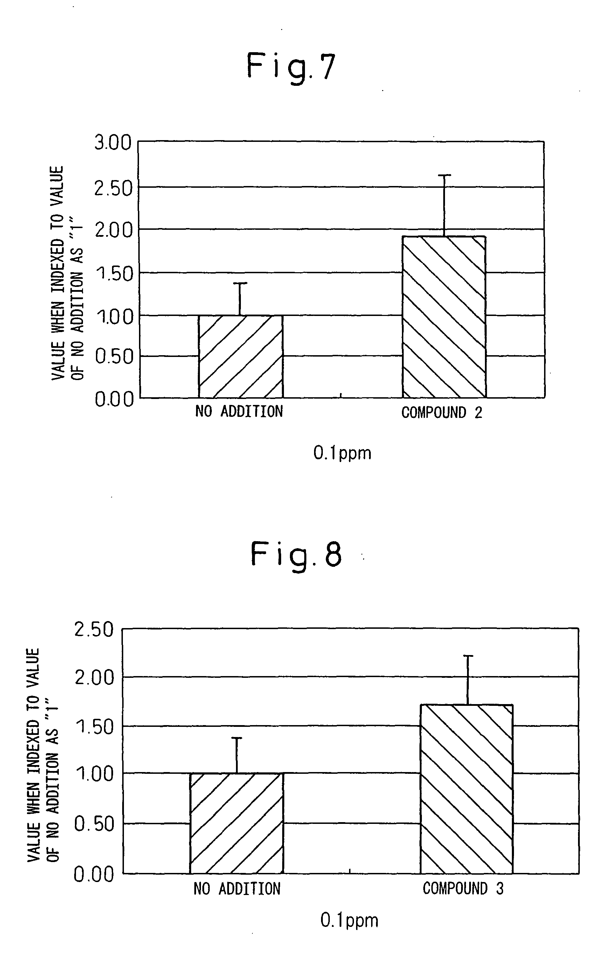

Using the average number of migrating cells in the negative control group as "1", the ratio of the number of migrating cells of each test substance added group with respect to this was calculated and used as an indicator of the melanocyte migration stimulation effect. These results are shown in FIGS. 6 to 8. Note that FIG. 6 shows the melanocyte migration stimulation effect of compound 1, FIG. 7 shows the melanocyte migration stimulation effect of compound 2, and FIG. 8 shows the melanocyte migration stimulation effect of compound 3.

From FIGS. 6 to 8, it became clear that not only do the compounds of the present invention promote melanocyte proliferation, but they also promote migration of melanocytes.

### Test Example 3: Human Anti-Hair Graying Effect Test

Furthermore, as a test of use of the anti-hair graying agent, a test of the anti-hair graying effect was performed on humans.

### 1. Preparation of Sample Solution

As the test substances, compounds 1, 2 and 3 were used. Each test substance was dissolved in a mixed solution of an aqueous 70% ethanol solution plus 1% 1,3-butylene glycol and the resultant mixture used as the sample (test substance concentration: 0.25%).

### 2. Test Method

The number of gray hairs among approximately 1000 hairs was counted before and after use of each sample. The changes of the ratios were used to compare the anti-hair graying actions. Specifically, each sample was applied on the scalps of 10 male subjects two times per day, 2 ml per time, continuously for six months. One thousand hairs of each of the subjects were counted divided into black hairs and gray hairs before the application and right after the end of six months after termination of the application to investigate the hair graying rate. The ratios (%) of the subjects where the hair graying rates after use declined 20% or more compared with before use, changed within ±20% or increased by 20% or more were respectively calculated. The results are shown in Table 1.

**Table 1**

| Test substance | Hair graying rate | | |
|---|---|---|---|
| | Declining 20% or more | Changing within ±20% | Increasing 20% or more |
| Compound 1 | 70% | 30% | 0% |
| Compound 2 | 60% | 30% | 10% |
| Compound 3 | 70% | 20% | 10% |

As clear from Table 1, the hair graying rate significantly declined by application of the compounds of the present invention. It was confirmed that the compounds were effective as anti-hair graying agents in the tests of actual use as well.

### Example 2

Examples of formulations of anti-hair graying agents including the compounds of the present invention will be shown below as Example 2. In each case, significant effects were obtained in the test of the proliferation stimulation effect shown in Test Example 1, the test of the migration stimulation effect shown in Test Example 2 and the test of the human anti-hair graying effect shown in Test Example 3.

### Formulation Example 1: O/W Milky Lotion Type Anti-Hair Graying Agent

| Ingredient | Formulating Amount (mass%) |
|---|---|
| (Phase A) | |
| POE (60) hydrogenated castor oil | 2.0 |
| Glycerin | 10.0 |
| Compound 1 | 1.0 |
| Dipropylene glycol | 10.0 |
| 1,3-butylene glycol | 5.0 |
| Polyethylene glycol 1500 | 5.0 |

| (Phase B) | |
|---|---|
| Cetyl isooctanate | 10.0 |
| Squalane | 5.0 |
| Vaseline | 2.0 |
| Propylparaben | 2.0 |

| (Phase C) | |
|---|---|
| 1% aqueous Carboxylvinylpolymer solution | 30.0 |
| Sodium hexametaphosphate | 0.03 |
| Ion exchanged water | 8.35 |

| (Phase D) | |
|---|---|
| Potassium hydroxide | 0.12 |
| Ion exchanged water | Bal. |
| Total | 100.0 |

### (Preparation Method)

Phase A and Phase B were respectively heated to dissolve at 60°C, mixed, and treated by a homo mixer to prepare a gel. Next, the dissolved Phase C was added thereto, and lastly the dissolved Phase D was added. The mixture was emulsified by a homo mixer to obtain an O/W milky lotion type of anti-hair graying agent.

### Formulation Example 2: Cream Type Anti-Hair Graying Agent

| Ingredient | Formulating Amount (mass%) |
|---|---|
| (Phase A) | |
| Liquid paraffin | 5.0 |
| Cetostearyl alcohol | 5.5 |
| Glyceryl monostearate | 3.0 |
| Compound 2 | 3.0 |
| Propylparaben | 0.3 |
| Perfume | 0.1 |

| (Phase B) | |
|---|---|
| Vitamin E succinate ester | 5.0 |
| Glycerin | 8.0 |
| Dipropylene glycol | 20.0 |
| Polyethylene glycol 4000 | 5.0 |
| Sodium decyl sulfate | 0.1 |
| Sodium hexametaphosphate | 0.005 |
| Ion exchanged water | Bal. |
| Total | 100.0 |

### (Preparation Method)

Phase A and Phase B were respectively heated to dissolve and mixed, then emulsified by a homo mixer to obtain a cream type anti-hair graying agent.

### Formulation Example 3: Hair Tonic

| Ingredient | Formulating Amount (mass%) |
|---|---|
| (1) Compound 3 | 3.0 |
| (2) 95% ethanol | 85.0 |
| (3) Propylene glycol | 1.0 |
| (4) Sucrose | 0.2 |
| (5) Vitamin E acetate | 1.0 |
| (6) Glycine | 0.001 |
| (7) Menthol | 1.0 |
| (8) POE (100) hydrogenated castor oil | 0.3 |
| (9) Isostearyl alcohol | 0.2 |
| (10) Eucalyptus oil | 0.2 |
| (11) Ion exchanged water | Bal. |
| (12) Succinic acid | 0.01 |
| (13) Perfume | q.s. |
| (14) Color | q.s. |
| Total | 100.0 |

### (Preparation Method)

(1), (3) to (5), (7) to (10), and (13) were dissolved in (2) (Phase A). (6), (12), and (14) were dissolved in (11) and mixed with Phase A to obtain the hair tonic.

### Formulation Example 4: Anti-Hair Graying Agent

| Ingredient | Formulating Amount (mass%) |
|---|---|
| Sodium N-cocolauryl-β-amino propionate | 0.2 |
| Compound 4 | 0.1 |
| Sodium dodecyl benzenesulfonate | 0.5 |
| Hydrogenated castor oil ethyleneoxide (40 mol) adduct | 1.0 |
| 95% ethanol | 54.0 |
| Ion exchanged water | Bal. |
| Total | 100.0 |

### (Preparation Method)

Ion exchanged water was added to 95% ethanol, then hydrogenated castor oil ethylene oxide (40 mol) adduct, sodium dodecyl benzenesulfonate and sodium N-cocolauryl-β-amino propionate were added to this, then compound 4 was added and the mixture was stirred to dissolve.

### Formulation Example 5: Lotion

| Ingredient | Formulating Amount (mass%) |
|---|---|
| (Phase A) | |
| Sorbitol | 3.0 |
| Glycerin | 5.0 |
| Resorcin | 0.02 |
| Ion exchanged water | Bal. |

| (Phase B) | |
|---|---|
| Compound 1 | 0.1 |
| POE (60) hydrogenated castor oil | 0.5 |
| 95% ethanol | 20.5 |
| Perfume | q.s. |
| Total | 100.0 |

### (Preparation Method)

Mix dissolving each ingredient of Phase A, adding the mixed solution of Phase B thereto, while agitating to make a homogeneous solution, the lotion was prepared.

### Formulation Example 6: Cream

| Ingredient | Formulating Amount (mass%) |
|---|---|
| (Phase A) | |
| Carnauba wax | 10.0 |
| Paraffin wax | 6.0 |
| Lanolin | 3.0 |
| Isopropyl myristate | 6.0 |
| Squalane | 8.0 |
| Liquid paraffin | 26.0 |
| POE sorbitan stearate | 2.0 |
| Sorbitan monostearate | 4.2 |
| Preservative | q.s. |

| (Phase B) | |
|---|---|
| Propylene glycol | 2.0 |
| POE (60) hydrogenated castor oil | 1.0 |
| Compound 2 | 0.1 |
| Purified water | Bal. |
| Total | 100.0 |

### (Preparation Method)

The ingredients of Phase A were mixed, then were heated to dissolve at approximately 75°C. The mixture of Phase B heated to 75°C was added thereto, while stirring, then the mixture was cooled until 45°C, while continuing stirring, then was allowed to stand to obtain a cream.

### Formulation Example 7: O/W Milky Lotion Type Anti-Hair Graying Agent

| Ingredient | Formulating Amount (mass%) |
|---|---|
| (Phase A) | |
| Compound 1 | 0.01 |
| POE (60) hydrogenated castor oil | 2.0 |
| Glycerin | 10.0 |
| Dipropylene glycol | 10.0 |
| 1,3-butylene glycol | 5.0 |
| Polyethylene glycol (molecular weight 1500) | 5.0 |

| (Phase B) | |
|---|---|
| Cetyl isooctanate | 10.0 |
| Squalane | 5.0 |
| Vaseline | 2.0 |
| Propylparaben | 2.0 |

| (Phase C) | |
|---|---|
| 1% aqueous Carboxylvinylpolymer solution | 30.0 |
| Sodium hexametaphosphate | 0.03 |
| Ion exchanged water | 8.35 |

| (Phase D) | |
|---|---|
| Ion exchanged water | 4.5 |

| (Phase E) | |
|---|---|
| Potassium hydroxide | 0.12 |
| Ion exchanged water | Bal. |
| Total | 100.0 |

### (Preparation Method)

Phase A and Phase B were respectively heated to dissolve at 60°C, mixed, then treated by a homo mixer to obtain a gel-like substance. Phase D was gradually added thereto, the mixture was dispersed by a homo mixer, then the dissolved Phase C was added, the dissolved Phase E was added, then the mixture was emulsified by a homo mixer to obtain an O/W emulsion.

### Formulation Example 8: Cream Type Anti-Hair Graying Agent

| Ingredient | Formulating Amount (mass%) |
|---|---|
| (Phase A) | |
| Compound 2 | 1.0 |
| Liquid paraffin | 5.0 |
| Cetostearyl alcohol | 5.5 |
| Glyceryl monostearate | 3.0 |
| POE (20)-2-octyldodecyl ether | 3.0 |
| Propylparabe | 0.3 |
| Perfume | 0.1 |

| (Phase B) | |
|---|---|
| Glycerin | 8.0 |
| Dipropylene glycol | 20.0 |
| Polyethylene glycol (molecular weight 4000) | 5.0 |
| Sodium hexametaphosphate | 0.005 |
| Ion exchanged water | Bal. |
| Total | 100.0 |

### (Preparation Method)

Phase A and Phase B were respectively heated to dissolve, mixed and emulsified by a homo mixer to obtain a cream.

### Formulation Example 9: Hair Tonic

| Ingredient | Formulating Amount (mass%) |
|---|---|
| Compound 1 | 10.0 |
| Peppermint (1,3-butylene glycol solution) | 0.1 |
| N,N-dimethyl-2-dodecylamine oxide | 1.0 |
| Hinokitiol | 1.0 |
| Vitamin B6 | 0.2 |
| Vitamin E acetate | 0.02 |
| Menthol | 0.2 |
| Evening Star (Swertia japonica) | 1.0 |
| Salicylic acid | 0.1 |
| Rosa rugosa (ethanol extract) | 0.5 |
| Propylene glycol | 2.0 |
| Sodium hyaluronate | 0.01 |
| POE (10) monostearate | 2.0 |
| 75% ethanol | Bal. |
| Total | 100.0 |

### (Preparation Method)

Each ingredient was sequentially added to the 75% ethanol and stirred to dissolve to obtain a hair tonic.

### Formulation Example 10: Hair Tonic

| Ingredient | Formulating Amount (mass%) |
|---|---|
| Compound 2 | 10.0 |
| Althea (ethanol extract) | 1.5 |
| Coix seed (ethanol extract) | 1.5 |
| N-N-dimethyl-2-tetradecylamine oxide | 0.05 |
| Hinokitiol | 1.0 |
| Vitamin B6 | 0.2 |
| Vitamin E acetate | 0.02 |
| Menthol | 0.2 |
| Salicylic acid | 0.1 |
| Puerariae Radix (ethanol extract) | 0.5 |
| Propylene glycol | 0.01 |
| Sodium hyaluronate | 0.01 |
| POE (10) monostearate | 2.0 |
| 70% ethanol | Bal. |
| Total | 100.0 |

### (Preparation Method)

Each ingredient was sequentially added to the 70% ethanol and stirred to dissolve to obtain a hair tonic.

### Formulation Example 11: Aerosol Anti-Hair Graying Agent

| Ingredient | Formulating Amount (mass%) |
|---|---|
| (Concentrated Solution Prescription) | |
| Compound 1 | 0.6 |
| 95% ethanol | 50.0 |
| Glycyrrhizinate | 0.1 |
| Althea (ethanol extract) | 0.05 |
| Peppermint (ethanol extract) | 0.05 |
| Evening Star (Swertia japonica) | 0.1 |

| | |
|---|---|
| Sodium lauryl sulfate | 0.1 |
| N-N-dihydroxymethyl-2-decylamine oxide | 0.2 |
| Hydrogenated castor oil ethyleneoxide (40 mol) adduct | 0.5 |
| Lactic acid | q.s. |
| Sodium lactate | q.s. |
| Fragrance | q.s. |
| Color | q.s. |
| Purified water | Bal. |
| Total | 100.0 |

| (Charged Formulation) | |
|---|---|
| Starting solution | 50.0 |
| Liquefied petroleum gas | 50.0 |

### (Preparation Method)

The starting solution formulation was dissolved, then was charged into a can, a valve was attached, then a gas was charged to obtain an aerosol preparation.

### Formulation Example 12: Lotion

| Ingredient | Formulating Amount (mass%) |
|---|---|
| (1) Compound 2 | 1.0 |
| (2) Ion exchanged water | Bal. |
| (3) Glycerin | 2.0 |
| (4) D-Erythritol | 0.5 |
| (5) POE sorbitan monooleate | 0.2 |
| (6) Monoammonium glycyrrhizinate | 0.03 |
| (7) 95% ethanol | 20.0 |
| (8) Cetyl octanoate | 0.05 |
| (9) Olive oil | 0.1 |
| (10) Citric acid | 0.01 |
| (11) Sodium citrate | 0.005 |
| (12) Perfume | q.s. |
| Total | 100.0 |

### (Preparation Method)

Part of (1), (3), (4) and (5) and (6) were dissolved in part of (2) (Phase A). The balance of (2), (8), (9) and the balance of (5) were mixed and treated by a homo mixer to obtain an emulsion. This was added to Phase A, then (10) and (11) were added and mixed and stirred, then (7) containing (12) was added to obtain a lotion.

### Formulation Example 13: Aerosol Anti-Hair Graying Agent

| Ingredient | | Formulating Amount (mass%) |
|---|---|---|
| (Starting Solution Formulation) | | |
| (1) | Compound 7 | 1.0 |
| (2) | 95% ethanol | 60.0 |
| (3) | Dipropylene glycol | 5.0 |
| (4) | Polyethylene glycol 1500 | 0.1 |
| (5) | Tocopherol acetate | 1.5 |
| (6) | Pantothenic acid alcohol | 0.5 |
| (7) | Menthol | 0.5 |
| (8) | PPG-20 Decyltetradeceth-10 | 0.1 |
| (9) | Ion exchanged water | Bal. |
| (10) | Talc | 0.2 |
| (11) | Lactic acid | 0.01 |
| (12) | Sodium lactate | 0.09 |
| (13) | Perfume | q.s. |
| (14) | Color | q.s. |
| Total | | 100.0 |

| (Charged Formulation) | | |
|---|---|---|
| Starting solution | | 70 |
| Dimethyl ether | | 30 |

### (Preparation Method)

(1), (3) to (8), and (13) were dissolved in (2) (Phase A). (11), (12), and (14) were dissolved in (9) and mixed with Phase A, then (10) was added to obtain a starting solution. The starting solution was charged into an aerosol can, a valve was attached, then dimethyl ether was added to obtain an aerosol anti-hair graying agent.

### Formulation Example 14: O/W Milky Lotion Type Anti-Hair Graying Agent

| Ingredient | Formulating Amount (mass%) |
|---|---|
| (Phase A) | |
| Polyoxyethylene (60 mol) added hydrogenated castor oil | 2.0 |

| | |
|---|---|
| Glycerin | 10.0 |
| Compound 4 | 1.0 |
| Dipropylene glycol | 10.0 |
| 1,3-butylene glycol | 5.0 |
| Polyethylene glycol 1500 | 5.0 |

| (Phase B) | |
|---|---|
| Cetyl isooctanate | 10.0 |
| Squalane | 5.0 |
| Vaseline | 2.0 |
| Propylparaben | 2.0 |

| (Phase C) | |
|---|---|
| 1% aqueous Carboxylvinylpolymer solution | 30.0 |
| Sodium hexametaphosphate | 0.03 |
| Ion exchanged water | 8.35 |

| (Phase D) | |
|---|---|
| Potassium hydroxide | 0.12 |
| Ion exchanged water | Bal. |
| Total | 100.0 |

### (Preparation Method)

Phase A and Phase B were respectively heated to dissolve at 60°C, mixed and treated by a homo mixer to obtain a gel. Next, the dissolved Phase C was added to this and lastly the dissolved Phase D was added, then the mixture was emulsified by a homo mixer to obtain an O/W milky lotion type of anti-hair graying agent.

### Formulation Example 15: Cream Type Anti-Hair Graying Agent

| Ingredient | Formulating Amount (mass%) |
|---|---|
| (Phase A) | |
| Liquid paraffin | 5.0 |
| Cetostearyl alcohol | 5.5 |
| Glyceryl monostearate | 3.0 |
| Compound 5 | 3.0 |
| Propylparaben | 0.3 |
| Fragrance | 0.1 |

| (Phase B) | |
|---|---|
| Vitamin E succinate ester | 5.0 |
| Glycerin | 8.0 |
| Dipropylene glycol | 20.0 |
| Polyethylene glycol 4000 | 5.0 |
| Sodium dodecyl sulfate | 0.1 |
| Sodium hexametaphosphate | 0.005 |
| Ion exchanged water | Bal. |
| Total | 100.0 |

### (Preparation Method)

Phase A and Phase B were respectively heated to dissolve, mixed and emulsified by a homo mixer to obtain a cream type anti-hair graying agent.

### Formulation Example 16: Anti-Hair Graying Agent

| Ingredient | Formulating Amount (mass%) |
|---|---|
| Compound 6 | 0.2 |
| Stearyl dimethylamine oxide | 0.5 |
| Hydrogenated castor oil ethyleneoxide (40 mol) adduct | 1.0 |
| 95% ethanol | 54.0 |
| Ion exchanged water | Bal. |
| Total | 100.0 |

### (Preparation Method)

The ion exchanged water was added to the 95% ethanol, then hydrogenated castor oil ethyleneoxide (40 mol) adduct and stearyl dimethylamine oxide were added thereto, compound 6 was then added and the resultant mixture was stirred to dissolve.

### Formulation Example 17: O/W Milky Lotion Type Anti-Hair graying Agent

| Ingredient | Formulating Amount (mass%) |
|---|---|
| (Phase A) | |
| POE (60)Hydrogenated castor oil | 2.0 |
| Glycerin | 10.0 |
| Compound 8 | 1.0 |
| Dipropylene glycol | 10.0 |
| 1,3-butylene glycol | 5.0 |
| Polyethylene glycol 1500 | 5.0 |

| (Phase B) | |
|---|---|
| Cetyl isooctanate | 10.0 |
| Squalane | 5.0 |
| Vaseline | 2.0 |
| Propylparaben | 2.0 |

| (Phase C) | |
|---|---|
| Carboxyl vinyl polymer 1% aqueous solution | 30.0 |
| Sodium hexametaphosphate | 0.03 |
| Ion exchanged water | 8.35 |

| (Phase D) | |
|---|---|
| Potassium hydroxide | 0.12 |
| Ion exchanged water | Bal. |
| Total | 100.0 |

### (Preparation Method)

Phase A and Phase B were respectively heated to dissolve at 60°C, mixed and treated by a homo mixer to obtain a gel. Next, the dissolved Phase C was added to this, then lastly the dissolved Phase D was added and the resultant mixture was emulsified by a homo mixer to obtain an O/W milky lotion type of anti-hair graying agent.

### Formulation Example 18: Anti-Hair Graying Agent

| Ingredient | Formulating Amount (mass%) |
|---|---|
| Compound 3 | 10.0 |
| Peppermint (1,3-butylene glycol solution) | 0.1 |
| N-N-dimethyl-2-dodecylamine oxide | 1.0 |
| Hinokitiol | 1.0 |
| Vitamin B6 | 0.2 |
| Vitamin E acetate | 0.02 |
| Menthol | 0.2 |
| Evening Star (Swertia japonica) | 1.0 |
| Salicylic acid | 0.1 |
| Rosa rugosa (ethanol extract) | 0.5 |
| Propylene glycol | 2.0 |
| Sodium hyaluronate | 0.01 |
| POE (10) monostearate | 2.0 |
| 99% ethanol | Bal. |
| Total | 100.0 |

### (Preparation Method)

Each ingredient was sequentially added to the 99% ethanol and stirred to dissolve to obtain an anti-hair graying agent.

## Claims

1. An anti-hair graying agent comprising, as an active ingredient, an aminocyclohexane derivative having the formula (1): 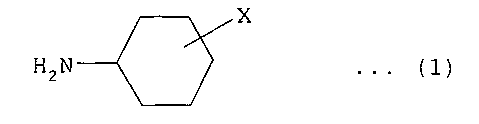 wherein X represents a -COOR group, where R represents an alkyl group or hydrogen atom, or an OH group, or a piperidine derivative having the formula (2): 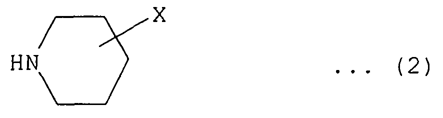 wherein X represents a -COOR group, where R represents an alkyl group or hydrogen atom or an OH group.

2. An anti-hair graying agent as claimed in claim 1, wherein said aminocyclohexane derivative is a 4-aminocyclohexane carboxylic acid derivative having the formula (3): 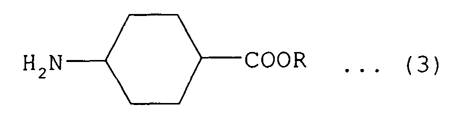 wherein R represents an alkyl group or hydrogen atom.

3. An anti-hair graying agent as claimed in claim 2, wherein said 4-aminocyclohexane carboxylic acid derivative is 4-aminocyclohexane carboxylic acid.

4. An anti-hair graying agent as claimed in claim 1, wherein said aminocyclohexane derivative is a 1-amino-1-cyclohexane carboxylic acid derivative having the formula (4): 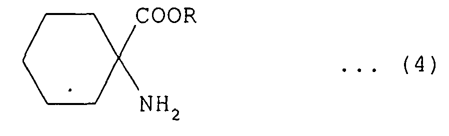 wherein R represents an alkyl group or hydrogen atom.

5. An anti-hair graying agent as claimed in claim 1, wherein said aminocyclohexane derivative is a 2-aminocyclohexane carboxylic acid derivative having the formula (5): 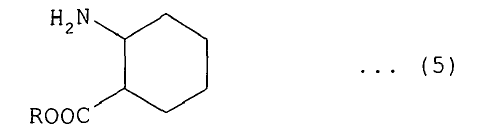 wherein R represents an alkyl group or hydrogen atom.

6. An anti-hair graying agent as claimed in claim 1, wherein said aminocyclohexane derivative is a 3-aminocyclohexane carboxylic acid derivative shown in formula (6): 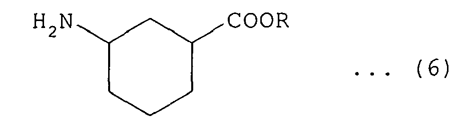 wherein R represents an alkyl group or hydrogen atom.

7. An anti-hair graying agent comprising the aminocyclohexane carboxylic acid derivative according to any one of claims 4 to 6, wherein R is a hydrogen atom, as an active ingredient.

8. An anti-hair graying agent as claimed in claim 1, wherein said aminocyclohexane derivative is a 4-am.inocyclohexanol having the formula (7):

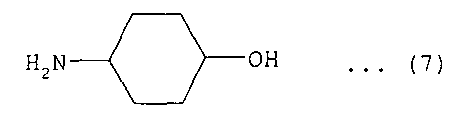

9. An anti-hair graying agent as claimed in claim 1, wherein said piperidine derivative is a piperidine carboxylic acid derivative having the formula (8): 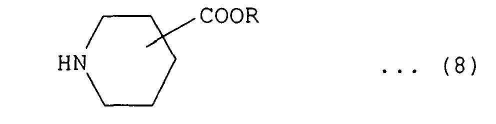 wherein R represents an alkyl group or hydrogen atom.

10. An anti-hair graying agent as claimed in claim 9, wherein said piperidine carboxylic acid derivative is 4-piperidine carboxylic acid.

11. An anti-hair graying agent as claimed in claim 9, wherein said piperidinecarboxylic acid derivative is 4-piperidine carboxylic acid ethyl ester.

12. An anti-hair graying agent as claimed in claim 1, wherein the piperidine derivative is the 4-hydroxypiperidine having the formula (9):
